# EUROPEAN PATENT APPLICATION

(11) **EP 2 749 318 A1**
(43) Date of publication of application: **02.07.2014**
(21) Application number: 12199678.9
(22) Date of filing: 28.12.2012
(51) Int. Cl.: A61N 7/02, A61B 18/00, A61B 18/02, A61B 18/12, A61B 18/18, A61B 18/20

(54) **Image-guided therapeutic apparatus and method of preparation of an image-guided therapeutic apparatus for treatment of tissue**

(71) Applicant: Theraclion SA, 92240 Malakoff (FR)
(72) Inventor: Lacoste, Francois, 94250 Gentilly (FR); Yon, Sylvain, 92220 Bagneux (FR)
(74) Representative: Hepp Wenger Ryffel AG

(57) **Abstract**

An image-guided therapeutic apparatus (1) comprises
- a treatment device (2), preferably a HIFU transducer, for treating tissue,
- at least one imaging device (3) for guidance of a treatment,
- means for providing an image (4),
- a display (5) for displaying an image and
- planning means (6) for planning a treatment.

The planning means (6) is adapted to create a lesion representation (9) of a lesion that will be created in tissue on the image (7) and to overlay said lesion representation (9) over said image (7). The size and/or shape and/or position of the lesion representation (9) is changeable, in particular in dependence on the characteristics of the tissue.

## Description

The present invention relates to an image-guided therapeutic apparatus and a method of preparation of an image-guided therapeutic apparatus for treatment of tissue according to the independent claims.

It is generally known to treat tissue non-invasively or minimal-invasively by high-intensity focussed ultrasound (HIFU) or radio-frequency ablation systems (RFA) or by cryotherapeutic devices or by laser or by microwave. Clinical procedures are typically performed in conjunction with an imaging procedure to enable treatment planning and targeting before applying a therapeutic or ablative level of energy to the tissue.

In US 2012/0150035 a method and an apparatus for selective treatment of tissue is disclosed. Before actual treatment an image of the treatment area is provided and the tissue is segmented into components. Depending on the tissue component areas are treated or are excluded from treatment. Such a system is not able to properly treat all areas within the region of interest of the image. In particular, the effect of the HIFU treatment may be smaller than anticipated.

It is an object of the present invention to avoid the disadvantages of the prior art and in particular to create an apparatus and a method enabling an optimized treatment planning within a region of interest of treatment of tissue.

The object is achieved by an apparatus and a method according to the independent claims.

In particular, the object is achieved by an image-guided therapeutic apparatus comprising a treatment device, preferably a HIFU-transducer, for treating tissue and at least one imaging device for guidance of a treatment. Furthermore, the apparatus comprises means for providing an image of a region to be treated and a display for displaying this image. Additionally, the apparatus comprises planning means for planning a treatment, wherein the planning means is adapted to create a virtual lesion representation of a lesion that will be created in tissue on this image and to overlay said lesion representation over said image. Characteristics of the lesion representation such as the size and/or the shape and/or the position of the lesion representation are changeable, in particular in dependence on the characteristics of the tissue.

Such an apparatus is able to adapt the lesion representation to the characteristics of the tissue and thereby treat different areas or types of tissues optimally. Hence, the apparatus becomes more efficient and safe.

The image on the display can be provided by an imaging device, such as the imaging device for guidance of the treatment or by any imaging device used in advance of the planning.

A lesion representation according to the invention is a virtual optical representation of the lesion that will be created by the treatment device.

The planning means can comprise a manual adjusting unit usable by an operator for changing the characteristics of the lesion representation.

A manual adjusting unit enables the operator to change the characteristics such as the size and/or the shape and/or the position of the lesion representation based on the image and the experience and/or knowledge of the operator. The treatment is thereby optimized and adapted to the actual region to be treated. In particular, differences in treatment between healthy and cancerous bone tissue or between different types of tissue can be made.

The planning means can also comprise an automatic image analysis unit to define and/or change and/or adapt the lesion representation of the treatment, preferably of the HIFU-treatment.

An automatic image analysis unit enables a fast and reliable determination of a lesion representation depending on the tissue within the image. The automatic image analysis unit can comprise image analysis software and preferably further prestored data on tissue detected.

The image analysis unit can determine the location of tissue and its characteristics such as its probable energy absorption coefficient, such as acoustic energy coefficient or thermal conductivity. Those values are used to size and position the lesion representation as close as possible to the predicted lesion thus helping optimize the treatment. A threshold function could be used to determine that a specific area of the image corresponds to a specific tissue type such as bone or blood vessel.

The apparatus can further be designed to allow a segmentation of tissue.

A segmentation of tissue is the fragmentation of the acquired image into image areas representing specific tissue types or organs, such as bone, skin, blood or vessels or glandular tissue or healthy and cancerous tissue. By such segmentation a clear allocation of tissue characteristics to specific image areas and a specific lesion representation for different areas becomes possible. Based on the specific lesion representation a specific treatment for those different areas also becomes possible.

The apparatus can be adapted to link the lesion representation created by the planning means with treatment characteristics, wherein the treatment characteristics comprise at least one of
- power
- pulse length
- acoustic frequency (in case of an acoustic treatment such as HIFU)
- repetition rate
- distance between pulses and position.

An adaptation of the above mentioned treatment characteristics enables an optimization of the treatment parameters based on the lesion representation.

The characteristics of the tissue can comprise at least one of
- thermal characteristics
- blood flow
- coefficient of absorption of energy
- tissue type
- volumetric blood perfusion.

Thermal characteristics can comprise conductivity or specific heat for example. The coefficient of absorption of energy comprises for example acoustic absorption coefficient in case of an HIFU-treatment or electrical resistance or impedance in case of a radio frequency ablation system.

The use of those characteristics of the tissue lead to an optimize treatment result.

The imaging device can be chosen from the group of
- Ultrasound transducer
- MRI (Magnetic resonance imaging) transducer
- X-Ray scanner
- Optical imaging devices.

Such an imaging device delivers accurate information over the tissue and the tissue types which is needed for accurate therapy.

The treatment device can be chosen from the group of
- HIFU (high intense focus ultrasound) transducer
- RFA (radio frequency ably system)
- Cryotherapeutic device
- Laser
- Microwave ablation system.

The imaging device and/or the image provided can be designed to provide an image containing three-dimensional data of region of interest.

The assessment of three-dimensional images leads to a better accuracy of the treatment.

The object is further achieved by a method of preparation of an image-guided therapeutic apparatus for treatment of tissue, preferably using an apparatus as described before, comprising the following steps:
- Providing an image of a region of interest
- displaying the image on a display
- analysing the image, preferably on the display, to detect characteristics of tissue in the region of interest
- overlaying a lesion representation over said image
- adjusting characteristics of said lesion representation within said apparatus, in particular based on detected characteristics of tissue of the analysed image.

This detection of the characteristics and the analysis of the image may be done automatically as a computer based analysis, semi-automatically as a computer assisted analysis or manually by the operator.

Such a method leads to a more accurate planning or a treatment which is better adapted to the tissue to be treated and by this leads to better treatment results.

The adjusted lesion representation can be linked with treatment characteristics comprising at least one of power and pulse length and acoustic frequency and repetition rate and distance between pulses and position of a pulse, preferably a HIFU pulse.

A link between the adjusted lesion representation and the treatment characteristics leads to an accurate reproduction of the lesion representation in the actual treatment as lesion.

The characteristics of the tissue can comprise at least one of
- thermal characteristics
- blood flow
- coefficient of absorption of energy
- tissue type
- volumetric blood perfusion
- location of a treatment area.

An adaptation of the lesion representation based on the above mentioned characteristics of the tissue leads to a more accurate and safe treatment.

The image of the region of interest can be taken before treatment and/or further images can be taken during treatment. It is in particular possible to use imaging methods such as e.g. PET, scintigraphy or to use image fusion combining the images of different imaging methods to infer the characteristics of the tissue to be treated.

The characteristics of the tissue influence the created lesions. Based on the tissue the lesion representation is hence chosen such as to optimally conform to the actually created lesion.

An image taken before treatment enables an accurate planning. Images acquired during the treatment enable the guidance of the treatment but may also allow the adaptation or an ongoing optimization of the planning of the treatment even during the treatment.

The tissue characteristics can be determined based on pre-stored data from a memory of the apparatus.

The tissue characteristics as physical values can be pre-stored and taken from a memory of the apparatus as data based on the image taken in the region of interest. This leads to an accurate planning and a safe treatment.

The image of the region of interest can be analysed automatically by an image analysis program so that the characteristics of the tissue can automatically be determined.

The use of an image analysis program and the automatic determination of the characteristics of the tissue lead to reproducible and accurate lesion representations and thereby to a safe and efficient treatment.

The extent and position of the lesion to be produced can be determined by means of simulation based on characteristics of tissue and on the treatment characteristics so that the characteristics of the lesion representation can be preferably automatically adapted to the results of the simulation.

A the lesion representation based on the simulation of a treatment leads to safer and more efficient treatments.

The simulation can be conducted by a thermal and/or acoustic simulation program which calculates the size and shape of the lesion representation from the parameters known such as tissue characteristics and treatment characteristics.

A location of the tissue can be determined three-dimensionally.

A three-dimensional determination of the tissue enables more accurate lesion representations and leads to safer treatments.

The size of a lesion representation created by a treatment device can be adjusted in a first step based on the characteristics of tissue and preferably in a second step the position of the lesion representation can be adjusted.

The adjustment of the lesion representation based on characteristics of tissue leads to more accurate lesion representations and safer and more efficient treatments. A further adjustment of the position enhances the safety of the treatment.

The Operator of the device may adjust the treatment with the help of the lesion representation. In particular treatment characteristics such as the power with which the treatment actuator is operated or the energy delivered by the treatment actuator to the tissue or the position of the actuator can be adjusted.

Such a treatment is more effective and safer for the patient and leads to a better reproducibility of the treatment.

In the following embodiments of the invention are described by means of figures. It is shown in
- Fig. 1: A schematic overview of an image-guided therapeutic apparatus
- Fig. 2: An ultrasound image of a region of interest
- Fig. 3: A schematic view of localisation of pulses
- Fig. 4a: An overview of variability of created lesion volume
- Fig. 4b: A photographic representation of lesions created in an experiment on ex vivo ox liver
- Fig. 5: A schematic view of a planned treatment.

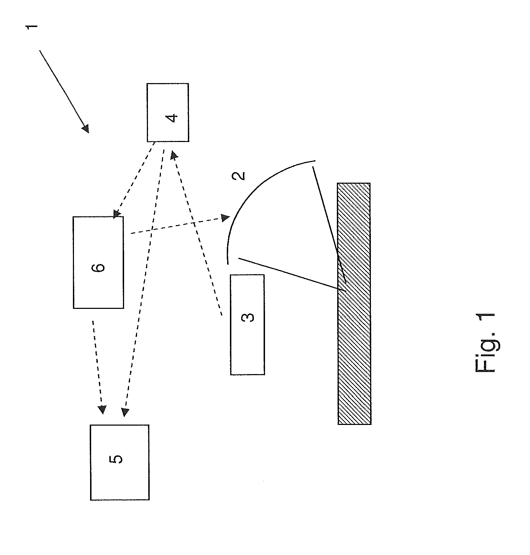

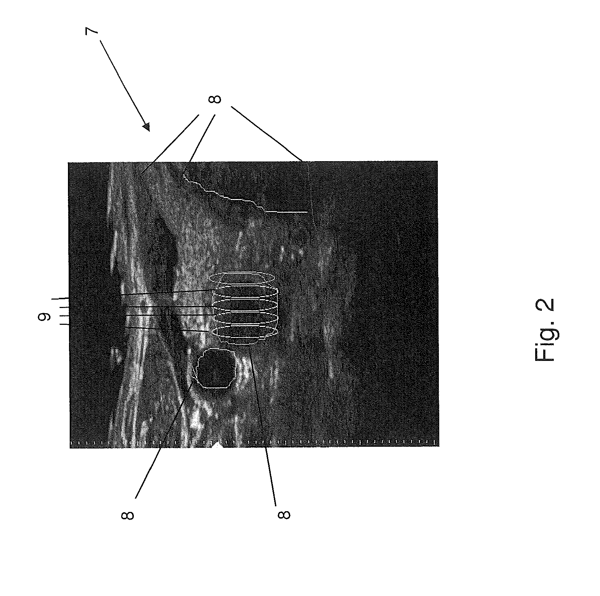

Fig. 1 shows a schematic overview of an apparatus 1 according to the invention. The apparatus 1 comprises a treatment device 2 and an imaging device 3. The treatment device 2 is adapted to conduct a HIFU-treatment of a region of interest in tissue. The imaging device 3 is adapted for guiding the treatment by ultrasound images. The apparatus further comprises means for providing an image 7 of tissue of the region of interest in a preliminary phase of the treatment. The image 7 can be taken by an imaging device 4 which is part of the apparatus 1. It is, however, also possible to use images taken previously in an imaging device separate from the apparatus. In this case the means for providing the image 7 are formed by a memory in the apparatus on which the images can be stored. The image 7 (see Fig. 2) can be displayed on display 5. The image 7, see Fig. 2 is segmented into different tissue types having specific tissue characteristics. The apparatus 1 further comprises planning means 6 such as a calculator for planning a treatment by overlaying a lesion representation 9 over the image on the display 5. The lesion representation can be adapted in size and/or shape and/or position depending on characteristics of the tissue in the region of interest.

Fig. 2 shows an ultrasound image 7 of a region of interest. The image 7 is segmented into different tissue types by segmentation lines 8. Lesion representations 9 are displayed within one tissue type. In this example the lesion representations take the shape of connected ellipses, displayed over the hypoechoic thyroid nodule to be treated by HIFU, each ellipse representing a single HIFU pulse.

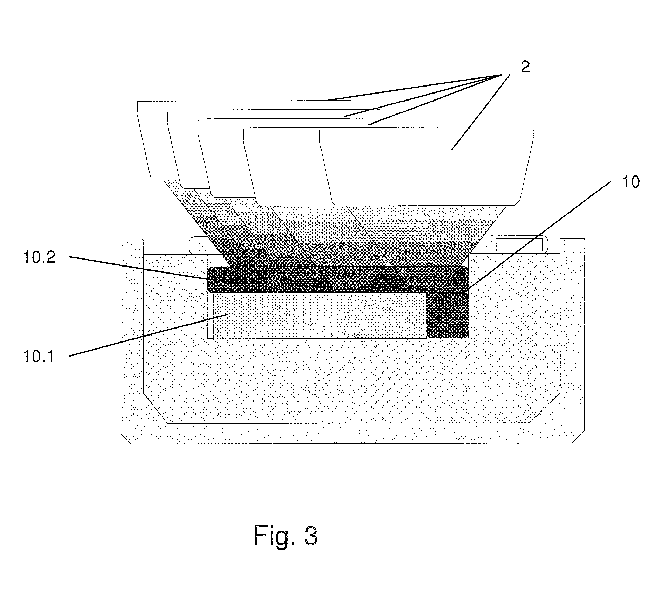

Fig. 3 shows a schematic view of different localisation of HIFU pulses in an experimental setting. A treatment device 2 treats tissue 10, comprising bone 10.1 and liver 10.2. The extent of the lesion created in the liver 10.2 varies with the distance of the focus to the bone 10.1 surface. Based on this finding, the adaptation of the lesion representation during the planning process based on the tissue type was developed.

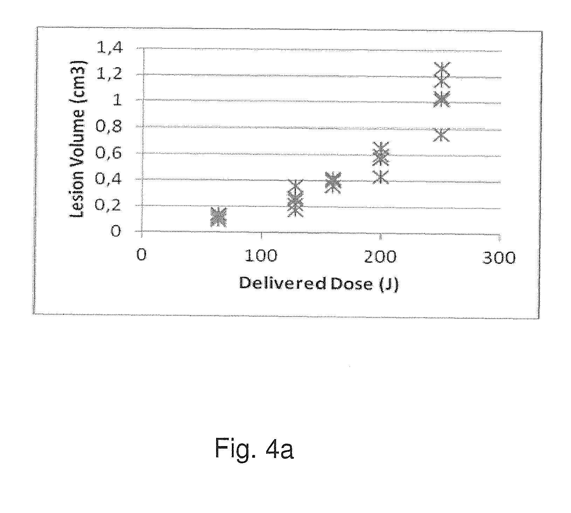

Fig. 4a shows an overview of the variability of created lesion volume based on different energy settings of the treatment device 2, see Figure 1. The graph is a result of a treatment of ex-vivo ox liver. It can be seen that the volume and the variability of the lesion increases with an increase of energy applied. Hence, the lesion representation overlaid with the image of the region of interest for planning of the treatment should be adapted based on the energy applied before treatment during a planning step.

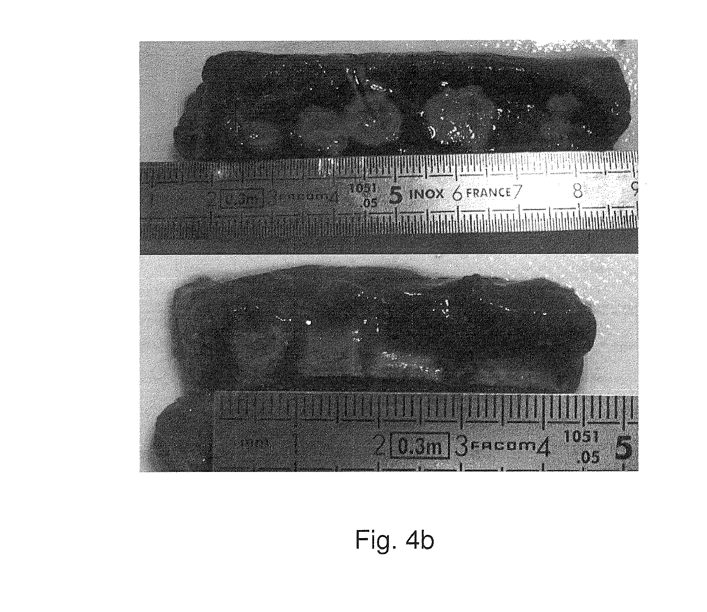

Fig. 4b shows experimental results giving an overview of the variability of created lesion volume based on different treatment depths (i.e. position of the focus with respect to the surface of the bone of the treatment device 2, see Figure 3). A HIFU treatment with a frequency of 3MHz, a power of 80W was carried out on an ox liver ex vivo. During HIFU delivery the bone was facing the coagulated tissue.

The lesions appear in brighter colour as compared to the untreated ex-vivo ox liver in dark colour. The top picture shows an end view of the treated tissue (i.e. the surface of the tissue in a plane perpendicular to the acoustic axis, facing the bone). The bottom picture shows a cut along a mid line of the first 4 lesions to estimate the depth of coagulation. The cut is made in a plane parallel to the acoustic axis, the scale in the bottom picture is located where the bone surface was during the experiment (c.f. also Fig. 3). It can be seen that the shape of the lesion changes with the position of the focus with respect to the bone surface. Hence, the lesion representation should be adapted based on the type of targeted tissue during a planning step.

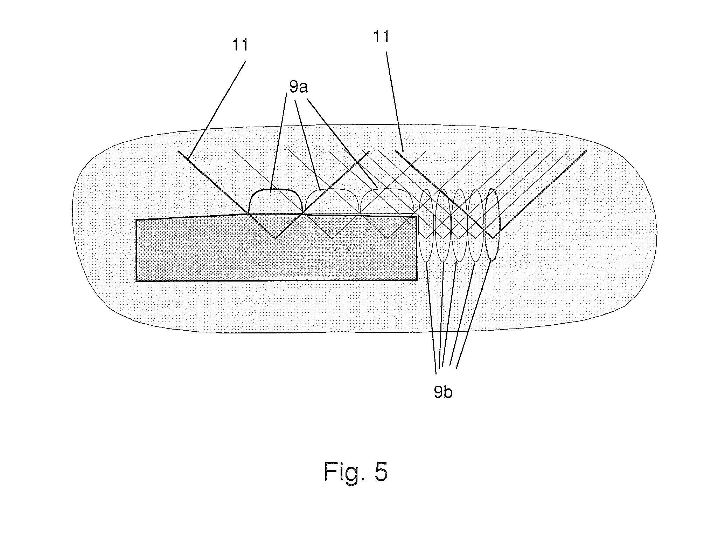

Fig. 5 shows a schematic view of a planned treatment. The treatment is conducted with a HIFU treatment device. The planned treatment is optimized by positioning lesion representations 9a, 9b. A "V" mark 11 indicating the acoustic cone created by the HIFU treatment device stays the same throughout the planned treatment, where the tip of the "V" represents the focus of the transducer. The lesion representations 9a, 9b differ depending on the tissue type treated. The lesion representation 9a is appropriate when the HIFU beam intercepts the bone surface, the focus being located 3 mm below that surface. Note that the lesion is wide, but not deep.

The lesion representation 9b represents the lesion obtained in soft tissue when a HIFU pulse is directed at a bone surface, the focus being 3 mm below that surface (see fig. 4b). The lesion representation 9b is deep but less wide. Through the complete procedure the focus of the HIFU treatment device remains at the same depth. The focus is positioned within the bone tissue under the bone surface. The lesion representation 9a has a broad shape in a direction perpendicular to the direction of propagation of the HIFU waves, while this lesion representation's 9a height is comparably low. The lesion representation 9b in soft tissue is shaped elliptical with a greater height but more narrow than the lesion representation 9a. The lesion representations 9b or 9a shapes will be overlaid on the image, depending on the type of tissue to be subjected to HIFU pulses. The size or shape of the lesion representation may be defined by the operator, e.g. by using a mouse or on a touch screen. It also may be defined by selecting a lesion representation out of a library of typical types of representations. The information relative to the type of tissue (i.e. soft tissue or bone surface) may be given by the operator, for example graphically, or automatically, using automatic detection of tissue type. Furthermore, the distance between single HIFU treatment pulses may be adapted to the tissue type. The distance Dₛ between single pulses in soft tissue is smaller compared to the distance D_{b} between single pulses in harder tissue, such as bone tissue. It is also conceivable to adapt the lesion representation if the region of interest comprises inhomogenities such as gas inclusions or if characteristics of the tissue change during the treatment, such as hypere-choic marks.

## Claims

1. An image-guided therapeutic apparatus (1) comprising
- a treatment device (2), preferably a HIFU transducer, for treating tissue,
- at least one imaging device (3) for guidance of a treatment,
- means (4) for providing an image (7),
- a display (5) for displaying said image (7) and
- planning means (6) for planning a treatment
- wherein the planning means (6) is adapted to create a lesion representation (9) of a lesion that will be created in tissue and to overlay said lesion representation (9) over said image (7),
**characterized in that** characteristics, in particular size and/or shape and/or position of the lesion representation (9) are changeable, in particular in dependence on the characteristics of the tissue.

2. Image-guided therapeutic apparatus (1) according to claim 1, **characterized in that** the planning means (6) comprises a manual adjusting unit useable by an operator for adjusting the lesion representation.

3. Image-guided therapeutic apparatus (1) according to any one of the preceding claims, **characterized in that** the planning means (6) comprise an automatic image analysis unit to define and analyze and adapt the lesion representation (9) of the treatment, preferably of the HIFU treatment.

4. Image-guided therapeutic apparatus (1) according to any one of the preceding claims, **characterized in that** the planning means (6) is adapted to create a segmentation of tissue.

5. Image-guided therapeutic apparatus (1) according to any one of the preceding claims, **characterized in that** the apparatus (1) is adapted to link the lesion representation (9) created by the planning means (6) with treatment characteristics, wherein the treatment characteristics comprise at least one of
- power
- pulse length
- duration of the energy delivery
- repetition rate
- distance between pulses and position
- acoustic frequency in case of acoustic treatments

6. Image-guided therapeutic apparatus (1) according to any one of the preceding claims, **characterized in that** the characteristics of the tissue comprise at least one of
- thermal characteristics
- blood flow
- coefficient of absorption of energy
- tissue type
- volumetric blood perfusion.
- coagulation temperature

7. Image-guided therapeutic apparatus (1) according to any one of the preceding claims, **characterized in that** the imaging device (3) is chosen from the group of
● Ultrasound scanner
● MRI (magnetic resonance imaging)
● X-ray scanner
● Optical imaging

8. Image-guided therapeutic apparatus (1) according to any one of the preceding claims, **characterized in that** the treatment device (2) is chosen from the group of
● HIFU (high intensity focussed ultrasound) transducer,
● RFA (radio frequency ablation system),
● Cryotherapeuthic device.
● Laser
● microwave ablation system

9. Image-guided therapeutic apparatus (1) according to any one of the preceding claims, **characterized in that** the imaging device (3) and/or the image provided is designed to provide an image (7) containing three-dimensional data of a region of interest.

10. A method of preparation of an image-guided therapeutic apparatus (1) for treatment of tissue, preferably using an apparatus as claimed in any one of claims 1 to 9 comprising the following steps:
● Providing an image (7) of a region of interest;
● Displaying the image (7) on a display (5);
● Analyzing the image (7), preferably on the display (5), to detect characteristics of tissue in the region of interest;
● Overlaying a lesion representation (9) over said image;
● Adjusting characteristics of said lesion representation (9) within said apparatus (1) of a treatment device, in particular based on detected characteristics of the tissue of the analysed image (7).

11. Method according to claim 10, **characterized in that** the adjusted lesion representation (9) is linked with treatment characteristics comprising at least one of
- power
- pulse length
- duration of the energy delivery
- repetition rate
- distance between pulses
- position of a pulse, preferably a HIFU pulse
- acoustic frequency in case of an acoustic treatment.

12. Method according to any one of claims 10 to 11, **characterized in that** the characteristics of the tissue comprise at least one of
- thermal characteristics
- blood flow
- coefficient of absorption of energy
- tissue type
- volumetric blood perfusion
- location of a treatment area
- coagulation temperature.

13. Method according to any one of claims 10 to 12, **characterized in that** the image (7) of the region of interest is taken before treatment and/or further images (7) are taken during treatment.

14. Method according to any one of the claims 10 to 13, **characterized in that** the tissue characteristics are determined based on prestored data from a memory of the apparatus (1).

15. Method according to any one of the claims 10 to 14, **characterized in that** the image (7) of the region of interest is analyzed automatically by an image analysis program and the characteristics of the tissue are automatically determined.

16. Method according to any one of the claims 10 to 15, **characterized in that** the treatment characteristics are determined by means of a simulation based on characteristics of tissue and the lesion representation (9), and preferably the treatment characteristics is automatically adapted to the result of the simulation.

17. Method according to any one of the claims 10 to 16, **characterized in that** a location of the tissue is determined three-dimensionally.

18. Method according to any one of the claims 10 to 17, **characterized in that** a size of a lesion representation (9) created by a treatment device (2) is adjusted in a first step based on the characteristics of tissue, preferably as a second step the position of the lesion representation (9) is adjusted.

19. Method according to any one of claims 10 to 18, **characterized in that** tissue is treated with a treatment transducer having adjusted treatment characteristics based on the lesion representation (9).
